# EUROPEAN PATENT SPECIFICATION

(11) **EP 2 817 275 B1**
(45) Date of publication and mention of the grant of the patent: **13.09.2023**
(21) Application number: 12869155.7
(22) Date of filing: 29.02.2012
(51) Int. Cl.: C07C 1/00

(54) **CONVERSION OF LIPIDS**
UMWANDLUNG VON LIPIDEN
CONVERSION DE LIPIDES

(30) Priority: 24.02.2012 CN 201210048253
(43) Date of publication of application: 31.12.2014
(73) Proprietor: Dalian Institute Of Chemical Physics, Chinese Academy Of Sciences, Liaoning 116023 (CN); BP P.L.C., London SW1Y 4PD (GB); Petrochemical Research Institute PetroChina, Beijing 100195 (CN)
(72) Inventor: TIAN, Zhijian, Dalian Liaoning 116023 (CN); WANG, Congxin, Dalian Liaoning 116023 (CN); WANG, Lei, Dalian Liaoning 116023 (CN); WANG, Bingchun, Dalian Liaoning 116023 (CN); YAN, Lijun, Beijing 100195 (CN); QU, Wei, Dalian Liaoning 116023 (CN); LI, Peng, Dalian Liaoning 116023 (CN); MA, Huaijun, Dalian Liaoning 116023 (CN); XU, Renshun, Dalian Liaoning 116023 (CN)
(74) Representative: CH Kilger Anwaltspartnerschaft mbB
(86) International application number: PCT/CN2012/071759
(87) International publication number: WO 2013/123678

(56) References cited:
- WO-A1-2008/152199
- US-A1- 2006 207 166
- US-A1- 2006 207 166
- KIKHTYANIN O V ET AL: "Hydroconversion of sunflower oil on Pd/SAPO-31 catalyst", FUEL, IPC SCIENCE AND TECHNOLOGY PRESS, GUILDFORD, GB, vol. 89, no. 10, 1 October 2010 (2010-10-01), pages 3085-3092, XP027136778, ISSN: 0016-2361 [retrieved on 2010-06-03]
- IKHTYANIN, OLEG V. ET AL.: 'Hydroconversion of Sunflower Oil on Pd/SAPO-31 Catalyst' FUEL vol. 89, no. ISSUE, 03 June 2010, pages 3085 - 3092, XP027136778

## Description

This invention relates to the conversion of organic materials, in particular to the conversion of lipids. Aspects of the invention relate to the production of a hydrocarbon product. In examples of the invention, a conversion process converts a lipid feedstock to form a hydrocarbon product. In preferred aspects of the invention, the hydrocarbon product includes alkanes. In examples, the product formed has high proportion of branched alkanes. Preferably, the product formed has a controlled proportion of branching. Preferably the product is suitable for use as, or as a component in middle distillate, including for example diesel fuels and/or jet fuels. In preferred examples of the invention, the lipids are obtained from a biological source.

In the present disclosure, reference is made to application of features of the invention to diesel fuels. It should be understood that, unless apparent to the contrary, the features may also be applied to other fuels, for example jet fuels.

Aspects of present invention disclose a process for producing biodiesel and/or bio-jet from lipids, where main components of the biodiesel comprise iso-paraffins.

Preferred aspects of the invention relate to the catalytic conversion of organic materials, for example lipids, preferably to form a product suitable for use in the preparation of a diesel fuel and/or a jet fuel.

It is generally accepted that there is a need for alternative sources of fuels to fossil fuels, both in relation to the ever increasing global demand for fuel, and environmental concerns in relation to the reliance on fossil fuels as an energy source. The production of non-fossil fuels is gaining significant interest. In particular, the production of fuel from renewable feedstock is now of significant interest, for example in relation to the development of bio-fuels which are generated using for example plant-based feedstock material such as sugarcane or corn. Biodiesel produced from a renewable resource such as vegetable oil and/or animal fat is another alternative energy source receiving interest.

In the so-called "first generation" of biodiesel fuels, examples of such fuels were based on fatty acid methyl ester (FAME) which was obtained from lipid and methanol via transesterification. Such fuels have been commercialized. There can be many potential drawbacks in its manufacture process.

For example, the feedstock for the production of FAME must normally contain a low content of free fatty acid and water, and the acid or base component generated in the manufacture process is harmful to environment. Furthermore, the main components of the first generation biodiesel are unsaturated esters with relatively low stability, low energy value, high viscosity and high freezing point. Thus, when the first generation biodiesel is used as an engine fuel, it must normally be blended with diesel derived from fossil fuel, often less than 20%, or even less than 10% of the fuel volume. The application and development of the first generation biodiesel has generally been limited to date.

In the second generation biodiesel, the biodiesel comprises diesel-range hydrocarbons derived from a lipid feedstock via hydrogenation and deoxygenation. The lipid feedstock comprises for example triglyceride, glyceride and/or fatty acid (and possibly other components). Examples of the renewable lipid feedstock typically have fatty acid carbon chains of 12-24 carbon atoms, mainly of 16-18 carbon atoms. When it is hydrogenated and deoxygenated under suitable reaction conditions, the product obtained is generally a mixture of hydrocarbons having the main components of C₁₁-C₂₄ paraffins.

There are many potential advantages of the second generation biodiesel as compared with the first generation biodiesel. Firstly, existing refineries can directly be utilized for producing the second generation biodiesel. Also, the composition of the second generation biodiesel is similar to that of the fossil fuel diesel, and the viscosity and energy value are close to those of the fossil diesel, moreover, the cetane value is much higher (greater than 70) and the sulfur content is much lower (below 10 ppm) compared with first generation biodiesel. Furthermore, since the main components of the second generation biodiesel are saturated hydrocarbons, the storage and transportation of the second generation biodiesel are very stable compared with for first generation biodiesel. In addition, in some cases, an early boiling fraction of the second generation biodiesel may be used in biojet fuel. In some examples, the biodiesel is substantially fungible so can be blended with existing refinery diesel and can use the same storage and distribution networks.

A desirable product of the hydrotreatment process for producing diesel-range fuel from lipid is C₁₁-C₂₄ iso-paraffin (branched alkane) with high cetane value and low freezing point. However, the catalyst used in the conventional hydrotreatment process for producing diesel-range paraffins from lipid is a NiMo or CoMo sulfide catalyst over which high amount of water is generated. The hydro-treating catalysts described make n-paraffins and iso-paraffins. Subsequently, to obtain improved cold flow properties, an isomerization catalyst or component is used. However, as described in European Patent No. 1396531, International Patent Application No. WO2009156452, International Patent Application No. WO2008058664 and International Patent Application No. WO2008113492, it has been found that the water generated by the hydrotreating catalysts has potential to poison the isomerizing catalyst which is also required to produce the product. Therefore, existing processes for producing second generation biodiesel often consist of two separate steps as illustrated in the following showing a process for producing second generation biodiesel from lipid in the two-step process.

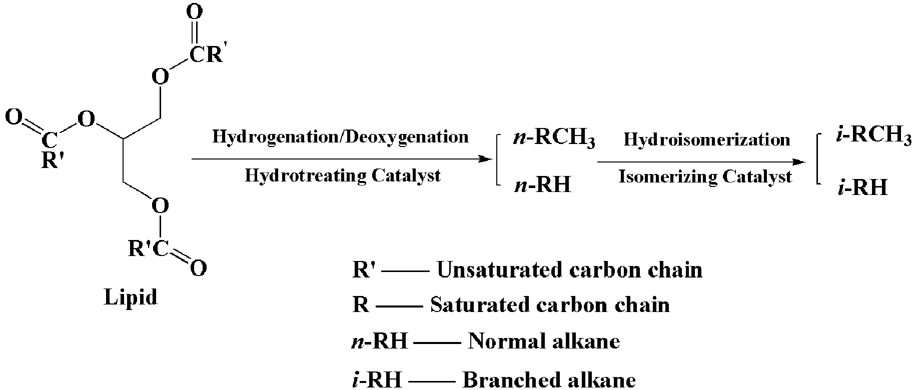

The general reaction process is as follows: the first step is a combined hydrogenation and deoxygenation step in which the lipid is hydrogenated and deoxygenated to produce normal-paraffins; the second step is a hydroisomerization step in which purified normal-paraffins are hydroisomerized for producing iso-paraffins. In more detail, in the first step, the lipid is saturated and deoxygenated to produce normal alkanes, water, propane, carbon monoxide, carbon dioxide and other byproducts in the hydrogenation and deoxygenation step. This step is described for example in US Patent Application No. 2008/0308457, US Patent No. 5705722, US Patent Application No. 2006/0186020, International Patent Application No. WO2006/075057. Then, in the second step, purified normal-paraffins are isomerized to form iso-paraffins in a hydroisomerizing step. This step is described for example in International Patent Application No. WO2010/028717, International Patent Application No. WO2009/156452, International Patent Application No. WO2008/113492 and US Patent Application No. 2010/000908.

The two-step process generally produces product having a high cetane value and low freezing point, and the low temperature performance of the product is good when it is used as diesel. However, the two-step process consists of multiple units in which the hydrogenation, deoxygenation or hydroisomerization proceeds individually over different catalysts. Thus the two-step process generally consumes a large amount of hydrogen and needs a relatively large investment for the manufacture of system equipment.

The hydrogen consumption and investment in the manufacture of devices would be reduced if the lipids could be converted to form paraffins, for example iso-paraffins, in a single-step process. For this to be achieved, the lipid would need to be converted to iso-paraffins through hydrogenation, deoxygenation and hydroisomerization simultaneously over a single catalyst in a single step.

Appl. Catal. A 329 (2007) 120-129, Green Chem. 12 (2010) 463-467 and Appl. Catal. A 375 (2010) 134-139, describes three pathways for converting triglycerides to normal-paraffins: hydrodeoxygenation, hydrodecarbonylation and hydrodecarboxylation.

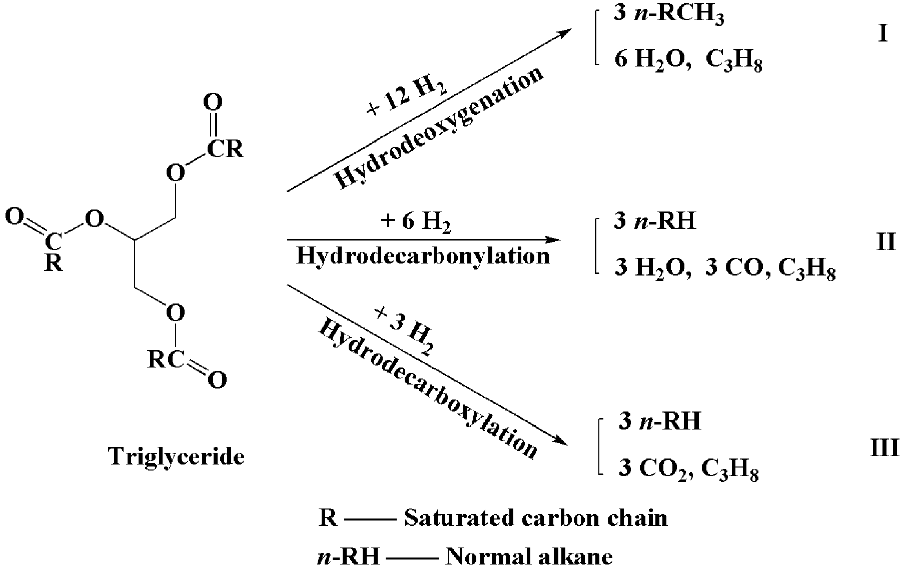

The oxygen atoms of the triglyceride are removed by releasing water, and normal-paraffin with the same carbon number of the fatty acid group is produced via hydrodeoxygenation as illustrated in pathway I. The carboxyl group of the triglyceride is removed by releasing carbon monoxide and water, and normal-paraffin with one carbon less than the fatty acid group is produced via hydrodecarbonylation as illustrated in pathway II. Also, the carboxyl group of the triglyceride is removed by releasing carbon dioxide, and normal-paraffin with one carbon less than the fatty acid group is produced via hydrodecarboxylation as illustrated in pathway III. In these three pathways, 6 molar quantities, 3 molar quantities and 0 molar quantities of water are produced, respectively. Therefore, when the lipid is converted to normal-paraffins through hydrodecarbonylation or hydrodecarboxylation pathways, the poison effect induced by the water to the catalyst will be reduced, and this can facilitate the process for producing iso-paraffins from lipid in a single-step.

It can also be identified from a consideration of the three pathways that the hydrogen consumption of the hydrodeoxygenation pathway is twice and four times of hydrodecarbonylation and hydrodecarboxylation pathways, respectively. Thus, when the reaction proceeds through hydrodecarbonylation or hydrodecarboxylation pathway, the hydrogen consumption will be reduced.

As reported in Appl. Catal. A 372 (2010) 199-208, the lipid conversion to paraffins proceeds through different pathways over different metal/γ-Al₂O₃ catalysts. The hydrodecarboxylation reaction is seen to be preferred over the Ni/γ-Al₂O₃ catalyst, while the hydrodeoxygenation is preferred over the Mo/γ-Al₂O₃ catalyst. The activity of the catalyst can also be improved when the loading amount of the active metal component increased as reported in Ind. Eng. Chem. Res. 45 (2006) 5708-5715 and Green Chem. 12 (2010) 463-467. The conversion of lipid and the selectivity of hydrodecarboxylation both increase over the 5 wt.% Pd/C catalyst as compared to those over the 1 wt.% Pd/C catalyst. In Micropor. Mesopor. Mater. 132 (2010) 174-180, a catalyst with highly dispersed palladium nanoparticles on the ultra-porous silica mesocellular foam has been reported. Over this catalyst, the selectivity of decarboxylation was seen to reach 100%. US2006/0207166 discloses a process for producing a fuel composition from vegetable and/or animal oil comprising hydrodeoxygenating and hydroisomerizing the oil in a single step.

A process for conversion of a lipid to a product including branched alkanes in a single step would be desirable.

In the present disclosure, the term lipid preferably includes, but preferably not exclusively includes, fatty acids, fatty acid derivatives, triglycerides, diglycerides, monoglycerides and phospholipids, fatty acid esters and/or a mixture of two or more of such compounds. Preferably the lipid compound or compounds are obtained from a biological source, either directly or indirectly. The lipid may for example include a plant derived fatty acid, for example a plant-derived fatty acid containing carbon chain unsaturation. The lipid may or may not be pure. For example, the lipid may include sugars and/or other components.

Aspects of the present invention find application in relation to the production of a hydrocarbon product from a feedstock containing a lipid, for example one or more fatty acid esters. The feedstock may include one or more other components.

According to the invention, there is provided a process for producing a hydrocarbon product, the process comprising contacting a feedstock with a catalyst composition in the presence of hydrogen, the feedstock including a lipid, and the catalyst composition being active for conversion of the lipid to the hydrocarbon product in a single step, wherein the catalyst composition effects hydrogenation, deoxygenation and hydroisomerization of the lipid, the catalyst composition comprising an M1-[Sup] catalyst, where M1 is an active metal and [Sup] comprises an acidic support, wherein the acidic support [Sup] comprises a zeolite and/or SAPO and further comprises a refractory inorganic oxide, wherein the amount of metal M1 present in the acidic support where M1 comprises cobalt, nickel, molybdenum or tungsten is between from about 1 to 30 wt.% based on the weight of the catalyst, wherein the acidic support comprises one or more selected from the group comprising SAPO-11, SAPO-41, ZSM-5, ZSM-22, ZSM-23, ZSM-48 or mixtures thereof, wherein the hydrocarbon product includes greater than 70 % measured by weight of C₁₁-C₂₄ alkanes based on the weight of the lipid, and the content of branched alkanes of the C₁₁-C₂₄ alkanes is greater than 60 % measured by weight of branched C₁₁-C₂₄ alkanes based on the weight of the C₁₁-C₂₄ alkanes.

Examples of the present invention provide a process in which, catalytically hydrogenating, deoxygenating and hydroisomerizing a lipid feedstock to paraffins may be carried out in a single step. Without wishing to be bound by any particular theory, the inventors have identified that oxygen atoms of the lipid in examples of the invention are mainly converted to CO and CO₂, and less are converted to H₂O. The single-step process of examples of the invention described herein may produce biodiesel with high cetane value, low freezing point, low aromatics and low sulfur. In examples of the invention the hydrogen consumption of the process may be less than that of the conventional two-step hydrogenation process.

The single-step process is schematically illustrated as follows:

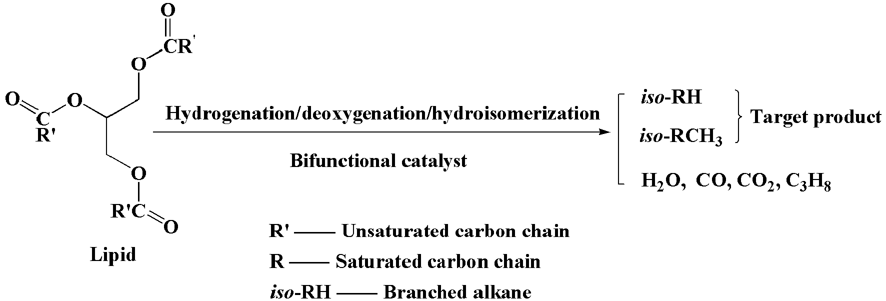

Without wishing to be bound by any particular theory, over the metal/acidic support catalyst, the oxygen atoms of lipid will mainly be removed by releasing carbon monoxide and water through hydrodecarbonylation and by releasing carbon dioxide through hydrodecarboxylation. Therefore, the process of examples of the invention produces less water than conventional processes. Water is potentially harmful to isomerizing active sites of the catalyst, and thus in examples of the present invention, the generated paraffins may be isomerized simultaneously over the same catalyst.

Furthermore, the hydrogen consumption of the present single-step process is less than that of the two-step hydrogenation process.

According to example processes of the invention, catalytically hydrogenating, deoxygenating and hydroisomerizing of the lipid feedstock to alkanes are carried out in a single step, for example over a single catalyst composition, for example in a fixed-bed reactor.

Without wishing to be bound by any particular theory, in examples, if the feedstock only comprises trilinolein, the maximum theoretical paraffins yield is determined to be 86.8wt.% assuming the trilinolein is converted to octadecane via hydrodeoxygenation, or 82.0wt.% assuming the trilinolein is converted to heptadecane via hydrodecarbolylation or hydrodecarboxylation pathway. Then if the feedstock only consists of tripalmitylglycerol, the maximum theoretical paraffins yield is 84. 1wt.% assuming the tripalmitylglycerol is converted to hexadecane via hydrodeoxygenation, or 78.9wt.% assuming the tripalmitylglycerol is converted to pentadecane via hydrodecarbonylation or hydrodecarboxylation.

According to examples of the present invention, catalytic hydrogenation, deoxygenation and hydroisomerization are carried out substantially simultaneously in the same reactor. Preferably there is no separation step within the reactor, for example to separate target products (for example iso-paraffins), intermediate products (for example normal-paraffins, organic oxygenates) and byproducts (for example CO, CO₂, C₃H₈, H₂O) in the process of the reaction.

Without wishing to be bound by any particular theory, it is thought that in examples of the invention, the oxygen atoms of lipid feedstock are mainly converted to CO and CO₂ through hydrodecarbonylation and hydrodecarboxylation, and less are converted to H₂O through hydrodeoxygenation. Thus, the poison effect induced by the water to the hydroisomerisation catalyst component may be reduced which can allow for the hydrogenation, deoxygenation and hydroisomerization of the feedstock to be combined into a single step.

Where reference is made herein to an acidic substrate, preferably the substrate is a Brønsted acid.

Examples described herein include description of methods for preparation of catalyst compositions. Some aspects of the invention extend to the case in which the catalyst composition is prepared using other methods or from different sources.

Preferably the catalyst is a multifunctional catalyst. Preferably the catalyst is active for catalytically hydrogenating, deoxygenating and hydroisomerizing the lipid feedstock. Preferably the catalyst is active to effect hydrogenation, deoxygenation and hydroisomerization of the lipid feedstock to paraffins in a single step.

Preferably the majority of the oxygen atoms of the lipid feedstock are converted to CO and CO₂ through hydrodecarbonylation and hydrodecarboxylation, the minority being converted to H₂O through hydrodeoxygenation.

Preferably the hydrodecarbonylation plus hydrodecarboxylation selectivity of the conversion reaction of the lipids is greater than 50%. Preferably the selectivity of the conversion reaction is determined from an analysis. Without wishing to be bound by any particular theory, it is believed that the ratio of C_{odd} to Cₑᵥₑₙ in the products is an indication as to the selectivity of the conversion reaction to hydrodecarbonylation plus hydrodecarboxylation. It is understood that the hydrodeoxygenation conversion reaction would generally produce a different length carbon chain alkane compared with the hydrodecarbonylation and hydrodecarboxylation reactions. In examples, one of the former pathway and the latter pathways would produce odd and even carbon backbone alkanes. By determining the ratio of C_{odd} to Cₑᵥₑₙ, a measure of the selectivity of the conversion reaction can be made. In examples, a hydrodeoxygenation conversion reaction would generally produce Cₑᵥₑₙ alkanes, where the hydrodecarbonylation and hydrodecarboxylation reactions produce C_{odd} alkanes.

Preferably the weight ratio of C_{odd} alkanes to Cₑᵥₑₙ alkanes in the hydrocarbon product is greater than one, where C_{odd} alkanes comprise an odd number of carbons, and Cₑᵥₑₙ comprise an even number of carbons.

Thus preferably the ratio of odd-numbered paraffins product to even-numbered paraffins product is greater than one.

Preferably the catalyst is a type of metal/acidic support solid bifunctional catalyst with high hydrogenation activity, high hydrodecarboxylation or hydrodecarbonylation activity, and high hydroisomerization activity. Over this catalyst, the C=C bonds of the lipid are saturated, the oxygen atoms of the lipid are removed, the paraffins are generated and isomerized.

The catalyst comprises a metal/acidic support solid catalyst. The catalyst may comprise a bifunctional catalyst.

The acidic support comprises a zeolite and/or SAPO, and a refractory inorganic oxide.

In preferred aspects of the invention, the acidic substrate comprises an M1-zeolite or an M1-silicoalumino phosphate (SAPO) catalyst (where M1 is a metal component).

Preferably the SAPO comprises a crystalline microporous silicoalumino phosphate composition. Silicoalumino phosphates are known to form crystalline structures having micropores which compositions can be used as molecular sieves for example as adsorbents or catalysts in chemical reactions. SAPO materials include microporous materials having micropores formed by ring structures, including 8, 10 or 12-membrered ring structures. Some SAPO compositions which have the form of molecular sieves have a three-dimensional microporous crystal framework structure of PO₂⁺, AlO₂⁻, and SiO₂ tetrahedral units. The ring structures give rise to an average pore size of from about 0.3 nm to about 1.5 nm or more. Examples of SAPO molecular sieves and methods for their preparation are described in US 4440871 and US6685905

Preferably the zeolite comprises a microporous aluminosilicate material.

Preferably the substituted aluminophosphates comprises a microporous crystalline material in which the Al and/or P is substituted for other elements, for example Si, Mg, Mn, Co, Zn, to form for example SAPO-n.

The acid support comprises one or more of the group comprising, SAPO-11, SAPO-41, ZSM-5, ZSM-22, ZSM-23, ZSM-48. The acidic support may comprise a mixture of two or more zeolites and/or SAPOs.

In some examples of the invention, the catalyst may include at least 70 wt.% of [Sup]. In some examples of the invention, the catalyst may include up to 30 wt.% of the metal.

The catalyst may include further components in addition to the metal M1 and acidic support [Sup].

The weight percent of metal M1 present in the acidic support where M1 comprises cobalt, nickel, molybdenum or tungsten is between from about 1 to 30 wt.%, preferably between from about 2 to 20 wt.% based on the weight of the catalyst.

Preferably the metal/acidic support solid catalyst is prepared using an impregnation method.

The acidic support [Sup] comprises a zeolite and/or SAPO and further comprises a refractory inorganic oxide.

In preferred examples of the invention, the acidic support is formed by mixing a zeolite and/or SAPO with a refractory inorganic oxide. Preferably the method of forming the acidic support includes the step of extruding and heating the mixture of zeolite/SAPO and refractory inorganic oxide. The heating step may include a calcination treatment. The heating treatment may for example proceed as follows: the extruded acidic support is preheated under oxygen or air atmosphere at between from about 100 to 150 °C for 1 to 4 h, and then it is calcined under oxygen or air atmosphere at between from about 300 to about 600 °C, for example between from about 500 to about 600 °C for 4 to 12 h. The resulting heat-treated mixture is preferably subsequently shaped.

Preferably the zeolite/SAPO support has been treated to alter its acidity, its pores and/or channels. Preferably a method or preparation of the acidic support includes carrying out an acid or alkali treatment.

Without wishing to be bound by any particular theory, preferably the pore diameter of the zeolite or SAPO is smaller than the molecular diameter of the reactants. For example, the pore diameter of SAPO-11 and ZSM-22 is about 0.40 nm x 0.65 nm and 0.46 x 0.57 nm, respectively, while the dynamic molecular diameter of the triglyceride in examples of the invention is greater than 0.6 nm. As a result, the reactants cannot generally access the inside of the micropores. Thus it is believed that the deoxygenation reaction can only be carried out on the outer surface of the zeolite crystals. However, the outer surface of the zeolite crystals is usually very small.

Without wishing to be bound by any particular theory, it is believed that an effect of the addition of the refractory inorganic oxide is to enhance the outer surface of the catalyst by building some hierarchical pores through which the reactants can access more active sites of the catalyst.

Preferably the refractory inorganic oxide is selected from the group comprising alumina and silica, or mixtures thereof.

Preferably the refractory inorganic oxide is present in an amount of between from about 20 to 60wt.% based on the weight of the catalyst.

In preferred examples, the product of the conversion is a biodiesel and/or component for use in a biodiesel and/or biojet. The product may undergo further processing and/or blending, for example to form a biodiesel product and/or a biojet product.

The majority of the hydrocarbon product is preferably C₁₁-C₂₄ paraffins, especially isomers of paraffins.

In examples of the invention, a target product is biodiesel and/or biojet. Thus preferably the product includes mainly paraffins with a number of carbon atoms ranging from 11 to 24, especially isomers. The yield of C₁₁-C₂₄ paraffins is in some preferred examples of the invention greater than 70wt.% based on the weight of the lipid feedstock. In preferred examples of the invention, the isomers content of C₁₁-C₂₄ paraffins is greater than 60wt.%.

Liquid product from the reaction may for example be analyzed by GC and/or GC-MS. Gas product from the reaction may be analyzed by GC.

Preferably, the components of the hydrocarbon product are mainly C₁₅-C₁₈ paraffins preferably with a yield of 60wt.% or more based on the lipid feedstock. Preferably the isomers content of C₁₅-C₁₈ paraffins is greater than 60wt.%. Preferably hydrocarbon product preferably comprises no more than 5wt.% C₆-C₁₀ paraffins and no more than 1wt.% cycloalkanes.

Preferably the lipid feedstock comprises a triglyceride, glyceride and/or fatty acid. Preferably the lipid is selected from the group comprising vegetable oil, animal fat, and waste oil or mixtures thereof.

The vegetable oil may be selected for example from the group comprising rapeseed oil, peanut oil, soybean oil, corn oil, rice oil, rice bran oil, safflower oil, palm oil, jatropha oil, castor oil, coconut oil, tall oil and olive oil or mixtures thereof. The animal fat may be selected for example from the group comprising lard oil, tallow oil, suet oil, chicken oil, fish oil and train oil or mixtures thereof. The materials for use as feedstock are not limited to refined oils. The feedstock may include waste oil and/or by-product of a processing operation, for example a vegetable oil processing operation producing a product having a high free fatty acid (FFA) content.

The lipid feedstock may be derived from any suitable source, preferably a biological source, for example a plant or animal source. In examples, the lipid feedstock includes components selected from the group consisting of vegetable oil, animal fat, waste oil, or mixtures thereof. The feedstock may include other components in addition to lipids.

The process will be operated at appropriate temperature and pressure. In examples of the invention, the preferred temperature of the process will be between from about 250 °C to 450 °C, for example 280 °C to 450 °C, for example 250 °C to 400 °C. Variations in temperature may occur across the catalyst, and preferably the average temperature across the catalyst is within that range of temperatures.

The pressure of the process may be for example between from 100 to 10000 kPa, for example between from 1000 to 10000 kPa, for example between from 2000 to 8000 kPa.

An appropriate ratio of hydrogen to feedstock will be chosen having regard to the lipid content. For example, in examples where the feedstock comprises an oil, the hydrogen/oil ratio may be in the range of from 300 to 3000 NL/L, for example between from 700 to 2000 NL/L.

An appropriate rate of fluid feedstock in the reactor will be chosen. In examples, the LHSV will be for example from about 0.1 to 5.0 h⁻¹.

In examples, the process includes catalytically hydrogenating, deoxygenating and hydroisomerizing the lipid feedstock to paraffins in a single step, at a temperature of between from about 250 to 400 °C, a pressure of between from about 1000 to 10000 kPa, a LHSV between from about 0.1 to 5.0 h⁻¹.and a hydrogen/oil ratio of between from about 300 to 3000 NL/L.

In some examples of the invention the conversion process itself does not include a separation stage, for example for separating normal-paraffins, iso-paraffins from water and/or other byproducts in the conversion process. In examples, there is substantially no separation of products during the conversion of lipid feedstock to paraffins.

Preferably the majority of, and preferably substantially all of any separation of products from the process is carried out after the conversion of the lipid to the hydrocarbon product. The produced paraffins may be subsequently subject to a separation process. For example, the paraffins product may be distilled into fractions, for example for biojet and biodiesel.

Preferably the catalytic process of hydrogenation, deoxygenation and hydroisomerisation are carried out substantially simultaneously in the same reactor. Preferably there is no separation step to separate target products, for example iso-paraffins, intermediate products, for example normal-paraffins, organic oxygenates, or by-products, for example CO, CO₂, C₃H₈, H₂O in the reaction process.

Preferably the process includes the step, after the conversion of the lipid to the hydrocarbon product, of separating the products.

Preferably the products are separated in one or more than one product separator after the single-step reaction.

Where hydrogen is to be recycled into the reactor, for example carbon dioxide may be separated from a recycle hydrogen stream. A purge may be carried out of hydrogen, propane, methane and/or carbon monoxide may be carried out for example to control levels of non-hydrogen components.

While it is envisaged that different reactor designs could be used in examples of the present invention, in some preferred examples, the reactor comprises a fixed-bed reactor. In examples, the reactor comprises a reactor system which may comprise one or more than one reactor element.

In examples of the invention, one or more than one type of catalyst can be loaded in the reactor or reactor element.

Thus the reactor of the present process may comprise a plurality of fixed-bed reactors in examples of the invention.

The catalytic conversion may be carried out in a reactor system in which the reactor system comprises one or more reactor elements. In some examples, the reactor or a reactor element of the system may contain a further catalyst composition. The further catalyst composition may comprise an M2-[Sup₁] catalyst, where M2 is an active metal and [Sup₁] comprises an acidic support. M2 may be the same as or different from M1. [Sup₁] may be the same as or different from [Sup].

The reactor system may comprise two or more reactor elements, the two or more reactor elements containing different catalyst compositions.

Thus one or more of the reactor beds may include more than one catalyst composition. Where more than one bed is present, the different beds may include the same or different catalysts, in any appropriate combination. Where reference is made to the conversion being carried out in a single step, the conversion may be carried out in a single reactor or reactor element, or across two or more reactors or reactor elements. In some examples, the conversion may be carried out over a single catalyst composition, or may be carried out over a plurality of catalyst compositions. In some examples, all of the catalyst compositions over which the conversion takes place may have a composition of M-[Sup], where M is preferably as described herein in relation to M1, and/or where [Sup] is preferably as described herein. The compositions of the catalysts may be the same or different.

In some examples, preferably the single step conversion is a conversion in which substantially no conversion products are removed until after the conversion of the lipid to the hydrocarbon product. Thus in a single step conversion, preferably there is substantially no separation of products during the conversion of lipid feedstock to paraffins.

In some examples, the single step conversion may or may not include the addition of compositions, streams or other material during the conversion. For example, a hydrogen-containing stream may be added to or upstream of a reactor, or to or upstream of a reactor element such that hydrogen is added during the single step conversion. In some examples, it will be preferred that substantially no addition of compositions, streams and/or other material is made during the conversion.

In some examples, reaction conditions may be changed during the single step conversion, for example with regard to temperature, pressure, space velocity, or other condition. In some examples, preferably there is substantially no change of one or more reaction conditions during the single step conversion, for example substantially no change in one or more of temperature, pressure, space velocity, during the single step conversion.

Preferably the feedstock contacts with the metal/acidic support solid bifunctional catalyst under hydrogen, hydrogen/nitrogen or hydrogen/inert gas atmosphere. Preferably the reaction conditions comprise one or more of the following: a temperature of 200 to 400 °C, a pressure of 1000 to 10000 kPa, a LHSV of 0.1 to 3.0 h⁻¹ and a hydrogen/oil ratio of 300 to 3000 NL/L, preferably a temperature of 280 to 400 °C, a pressure of 2000 to 8000 kPa, a LHSV of 0.4 to 1.5 h⁻¹ and a hydrogen/oil ratio of 700 to 2000 NL/L.

Preferably downstream of the reactor, a hydrogen-rich stream is separated from the products and hydrogen is returned to the reactor.

Advantages of some examples of the single-step process of the invention may include:
1. The hydrogenation, deoxygenation and hydroisomerisation of the feedstock may be combined by a single step by increasing the selectivity of hydrodecarboxylation and hydrodecarbonylation.
2. A product having a cetane value greater than 70, a freezing point below 0 °C and a sulfur content less than 10 ppm may be produced in some examples.
3. The hydrogen and energy consumption may be reduced in some single-step processes as compared with at least some of the existing two-step process.
4. The related process may provide in some cases a new strategy for producing fungible energy from renewable resources.
5. The capital cost for a plant using a single step process may be lower than the capital cost for a plant using a multi-step process.

There is provided a catalyst composition for use in a process for producing a hydrocarbon product from a feedstock including a lipid, the catalyst composition comprising an M1-[Sup] catalyst, where M1 is an active metal and [Sup] comprises an acidic support, wherein the acidic support includes a zeolite and/or SAPO, and a refractory inorganic oxide.

Preferably, the weight percent of metal M1 present in the acidic support where M1 comprises cobalt, nickel, molybdenum or tungsten is between from about 2 to 20 wt.% based on the weight of the catalyst.

Also provided is a method of forming a catalyst composition for use in a process for producing a hydrocarbon product from a feedstock including a lipid, the method including the steps of mixing a zeolite and/or SAPO with a refractory inorganic oxide to form an acidic support mixture, and forming the mixture into a support, applying a metal M1 to the support to form the catalyst composition.

Preferably the step of forming the mixture into a support includes the step of extruding and heating the mixture. The resulting heat-treated mixture is preferably subsequently shaped.

Preferably the metal M1 is applied to the support using an incipient wetness method.

Also provided is an apparatus for use in a process for producing a hydrocarbon product from a feedstock including a lipid, the apparatus including a reactor containing a catalyst composition comprising an M1-[Sup] catalyst, where M1 is an active metal and [Sup] comprises an acidic support, and further including a separator downstream of the reactor.

According to preferred examples of the invention, there is no separation of products or by-products of the reaction upstream of the said separator. The separator may comprise one or more separator units.

The reactor may comprise a reactor system, the reactor system including one or more reactor elements. A further catalyst composition may be contained in the reactor or a reactor element of the system. The further catalyst composition may comprise an M2-[Sup₁] catalyst, where M2 is an active metal and [Sup₁] comprises an acidic support. M2 may be different from M1. [Sup₁] may be different from [Sup]. The reactor system may comprise two or more reactor elements, the two or more reactor elements containing different catalyst compositions.

The inventors have identified that reducing strong Lewis acid sites on the acidic support, by modulating the acidity of, for example the molecular sieve, is helpful for preparing a preferred catalyst for use in aspects of the invention, over which hydrodeoxygenation will be inhibited and the selectivity of hydrodecarbonylation plus hydrodecarboxylation reactions will increase.

Thus it has been identified that designing and preparing the support material with suitable acidity, pores and channels, together with modulating the type and the amount of loading metal M1 can enable the preparation of a bifunctional catalyst with relatively high hydrodecarbonylation, hydrodecarboxylation and hydroisomerization activity. For example, the support material preferable has weak or medium acidity. Preferably the support material is a less strong Lewis acid and a more strong Brønsted acid. In some examples, the support material has pores extending substantially in one dimension. For example, the support material may have substantially one-dimensional pores having an axis between from 3.0 to 7.0 Å. More generally, preferably the pore diameter is between from 3.0 to 7.0 Å. Over such a catalyst, oxygen atoms of a lipid may in examples mainly be removed through hydrodecarbonylation and hydrodecarboxylation, and the generated paraffins will be hydroisomerized substantially simultaneously. A single-step process of directly converting the lipid to iso-paraffins with high cetane value, low freezing point, low aromatics and low sulfur may thus be achievable in some examples.

The invention extends to methods, compositions and/or apparatus substantially as herein described preferably with reference to the accompanying figures.

Preferred features of the present invention will now be described, purely by way of example having reference to the following figures:
Figure 1 shows schematically an example of apparatus for use in a process scheme for a single-step process for the production of a biodiesel;

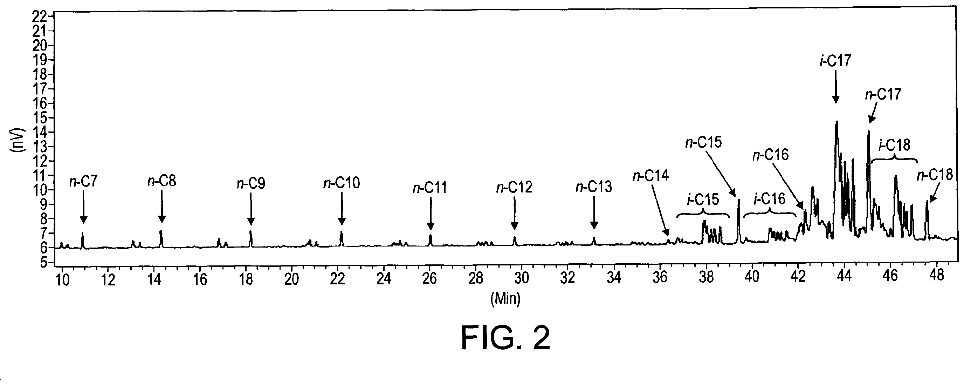

Figure 2 shows liquid product distribution analysis by GC-MS for the product of Example 2

The following examples illustrate aspects and features of the present invention but are not intended to limit the scope of the invention.

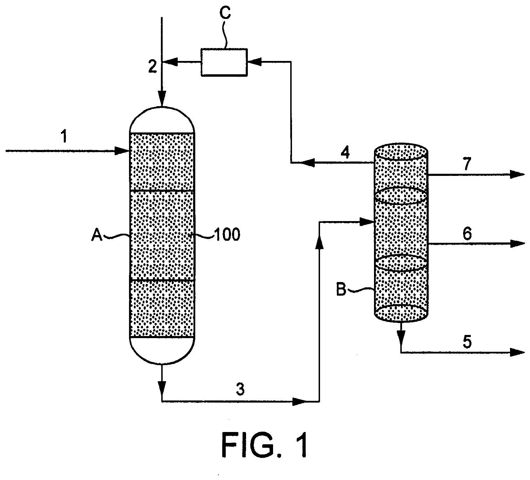

Figure 1 shows schematically an example of apparatus for use in a process scheme for a single-step process for the production of a biodiesel. The apparatus includes three units A, B and C as illustrated in Figure 1. It will be understood that practical examples of apparatus of the type illustrated in Figure 1 may include additional units or other elements. The apparatus includes a fixed-bed reactor A filled with catalyst 100. A feed stream 1 is fed to the reactor A together with a recycled hydrogen-containing stream 2. The feedstream 1 including the feedstock with hydrogen from the hydrogen-containing stream 2 over the catalyst 100 in the reactor A and undergoes hydrogenation, deoxygenation and hydroisomerization substantially simultaneously. A product stream 3 is generated, the product stream including iso-paraffins, normal-paraffins, water, propane, carbon dioxide, and carbon monoxide. The product stream 3 is passed from the base of the reactor A to a liquid product separator B, in which a diesel-range paraffin-containing stream 6, a gasoline-range paraffin-containing stream 7 and a water-containing stream 5 are separated from the product stream 3. A gaseous product stream 4 is fed from the liquid product separator B to a gas separator C. In the gas separator C, a hydrogen-containing stream 2 is generated and fed to the reactor A, by which method hydrogen is recycled back to the reactor A.

It will be understood that the process scheme shown in Figure 1 is an example of a possible apparatus, and different arrangements are possible.

### Example 1

### Catalyst Preparation

SAPO-11 and ZSM-22 zeolite were synthesized according to the directions of Flanigen et al. (Pure Appl. Chem. 58 (1986) 1351-1358) and Kokotailo et al. (Zeolites 5 (1985) 349-351). The synthesized zeolite was blended with 30wt/% γ-Al₂O₃ (produced by Shandong Aluminium Industry Co., Ltd.) and the mixture was extruded and calcined before being used as the support. All of the related catalysts were prepared by incipient wetness impregnation method to prepare the compositions indicated in the examples below.

The incipient wetness impregnation method is a known method for impregnating catalyst supports. It comprises for example the steps of adding a solution of catalyst metal M1 for example as a water soluble salt to a support in such a manner that the support remains dry in behaviour. The liquid is taken up into the pores of the support and preferably does not form a significant film on the outside of the catalyst. Subsequent removal of the solvent with, for example vacuum or nitrogen and/or heating leaves the catalyst precursor predominately in the pores.

After the addition of the metal M1, a calcination treatment may be carried out. For example, the calcination treatment may include heating to a temperature of between from 450 to 800 °C for 2 to 24 h under oxygen or air atmosphere, for example between from 500 to 600 °C for 4 to 12 h under oxygen or air atmosphere. In other examples, a lower temperature, for example between from 300 to 800 °C might be used.

Other appropriate methods could be used to prepare the catalysts in some examples. For example, an ion exchange method could be used.

### Comparative example

The following describes a two-step process for producing second generation biodiesel and/or biojet from soybean oil.

Refined soybean oil (produced by China Oil & Foodstuffs Corporation (COFCO), third grade, up to National Standard GB1535-2003) with a fatty acid composition of 0.1% lauric, 0.1% myristic, 10.2% palmitic, 3.7% stearic, 22.8% oleic, 53.7% linoleic and 8.6% linolenic and an acid value of 0.2 mgKOH.g⁻¹ (as measured by ASTM D974) was fed to a fixed-bed reactor (10 mm i.d. (internal diameter) and 600 mm in length) with a 5wt.%Ni-5wt.%Mo/γ-Al2O3 catalyst (7.8 g, 10 mL, 10-20 meshes). The reaction was operated at a LHSV (liquid hourly space velocity determined as the volume of liquid feed/h divided by the volume of the catalyst) of 0.5 h⁻¹, 355 °C, 4.0 MPa and a H₂/oil ratio of 1765 NL/L. After 6 hours, the gas product was collected and analyzed by GC-TCD (gas chromatography - thermal conductivity detector), while the liquid product was collected and analyzed by GC (gas chromatography) and GC-MS (gas chromatography - mass spectrometry).

The results were as follows: the soybean oil conversion was 100%, the hydrodecarbonylation plus hydrodecarboxylation selectivity was 28.8%, and the ratio of odd-numbered paraffins product to even-numbered paraffins product (determined using GC-MS) was 0.4. The liquid product contained 13.8wt.% of water and 86.2wt.% of organic product wherein the content of iso-paraffins was 1.7wt.% and the content of normal-paraffins was 97.3wt.%.

The liquid product was separated, and the purified product consisting of paraffins was obtained.

The purified product consisting of paraffins was fed to a fixed-bed reactor (10 mm i.d. and 600 mm in length) with a 0.5wt.%Pt/SAPO-11 catalyst (8.2 g, 10 mL, 10-20 meshes). The reaction was operated at a LHSV of 1 h⁻¹, 365 °C, 4.0 MPa and a H₂/oil ratio of 1353 NL/L. After 3 hours, the gas product was collected and analyzed by GC-TCD, while the liquid product was collected and analyzed by GC and GC-MS.

The results were as follows: the conversion was 84.5%, the yield of C₁₁-C₂₄ paraffins was 80.2wt.% based on the weight of the lipid feedstock, and the isomers content of C₁₁-C₂₄ paraffins was 86.2wt.%. The results are listed in Table 1.

### Comparative Example 2

A single-step process for producing second generation biodiesel and/or biojet from soybean oil over a Pt/SAPO-11 catalyst is described.

Refined soybean oil (produced by COFCO, third grade, up to National Standard GB1535-2003) was fed to a fixed-bed reactor (10 mm i.d. and 600 mm in length) with a 1wt.%Pt/SAPO-11 catalyst (7.5 g, 10 mL, 10-20 meshes). The reaction was operated at a LHSV of 1 h⁻¹, 357 °C, 4.0 MPa and a H₂/oil ratio of 1765 NL/L. After 3 hours, the gas product was collected and analyzed by GC-TCD, while the liquid product was collected and analyzed by GC and GC-MS.

The results were as follows: the soybean oil conversion was 100%, the hydrodecarbonylation plus hydrodecarboxylation selectivity was 70.6%, and the ratio of odd-numbered paraffins product to even-numbered paraffins product was 2.4. The yield of C₁₁-C₂₄ paraffins was 81.4wt.% based on the weight of the lipid feedstock, and the isomers content of C₁₁-C₂₄ paraffins was 86.2wt.%. The results are listed in Table 1 and the liquid product distribution is illustrated in Figure 1.

### Comparative Example 3

A single-step process for producing second generation biodiesel and/or biojet from soybean oil over a Pt/SAPO-11 catalyst is described.

Refined soybean oil (produced by COFCO, third grade, up to National Standard GB1535-2003) was fed to a fixed-bed reactor (10 mm i.d. and 600 mm in length) with a 1 wt.%Pt/SAPO-11 catalyst (8.1 g, 10 mL, 10-20 meshes). The reaction was operated at a LHSV of 0.6 h⁻¹, 339 °C, 6.0 MPa and a H₂/oil ratio of 1765 NL/L. After 3 hours, the gas product was collected and analyzed by GC-TCD, while the liquid product was collected and analyzed by GC and GC-MS.

The results were as follows: the soybean oil conversion was 99.2%, the hydrodecarbonylation plus hydrodecarboxylation selectivity was 57.5%, the yield of C₁₁-C₂₄ paraffins was 79.8wt.%, and the isomers content of C₁₁-C₂₄ paraffins was 81.5wt.%. The results are listed in Table 1.

### Comparative Example 4

A single-step process for producing second generation biodiesel and/or biojet over a Pt/SAPO-11 catalyst is described.

Refined soybean oil (produced by COFCO, third grade, up to National Standard GB1535-2003) was fed to a fixed-bed reactor (10 mm i.d. and 600 mm in length) with a 0.5 wt.% PT/SAPO-11 catalyst (8.1g, 10 mL, 10-20 mesh). The reaction was operated at a LHSV of 0.6 h⁻¹ , 364 °C, 6.0 MPa and a H₂/oil ration of 1353 NL/L. After 3 hours, the gas product was collected and analyzed by GC-TCD, while the liquid product was collected and analyzed by GC and GC-MS.

The results were as follows: the soybean oil conversion was 100%, the hydrodecarbonylation plus hydrodecarboxylation selectivity was 50.3%, the ratio of odd-numbered paraffins product to even-numbered paraffins product was 1.0, the yield of C₁₁-C₂₄ paraffins was 77.5 wt.% and the isomers content of C₁₁-C₂₄ paraffins was 73.9 wt.%. The results are listed in Table 1.

### Comparative Example 5

A single-step process for producing second generation biodiesel and/or biojet from soybean oil over Pd/SAPO-11 catalyst is described.

Refined soybean oil (produced by COFCO, third grade, up to National Standard GB1535-2003) was fed to a fixed-bed reactor (10 mm i.d. and 600 mm in length) with a 1wt.%Pd/SAPO-11 catalyst (8.7 g, 10 mL, 10-20 meshes). The reaction was operated at a LHSV of 0.6 h⁻¹, 350 °C, 6.0 MPa and a H₂/oil ratio of 1353 NL/L. After 3 hours, the gas product was collected and analyzed by GC-TCD, while the liquid product was collected and analyzed by GC and GC-MS.

The results were as follows: the soybean oil conversion was 99.0%, the hydrodecarbonylation plus hydrodecarboxylation selectivity was 52.7%, and the ratio of odd-numbered paraffins product to even-numbered paraffins product was 1.1. The yield of C₁₁-C₂₄ paraffins was 79.6wt.%, and the isomers content of C₁₁-C₂₄ paraffins was 66.2wt.%. The results are listed in Table 1.

### Comparative Example 6

A single-step process for producing second generation biodiesel and/or biojet from coconut oil over a Pt-Pd/SAPO-11 catalyst is described.

Refined coconut oil (produced by Southseas Oils & Fats Industrial (Chiwan) Limited, up to National standard NY/T230-2006) with a fatty acid composition of 46.5% lauric, 19.2% myristic, 9.8% palmitic, 3.0% stearic, 6.9% oleic and 2.2% linoleic and an acidic value of 0.1 mg_{KOH}.g⁻¹ was fed to a fixed-bed reactor (10 mm i.d. and 600 mm in length) with a 0.5wt.%Pd-0.5wt.%Pt/SAPO-11 catalyst (7.9 g, 10 mL, 10-20 meshes). The reaction was operated at a LHSV of 1 h⁻¹, 357 °C, 4.0 MPa and a H₂/oil ratio of 1765 NL/L. After 3 hours, the gas product was collected and analyzed by GC-TCD, while the liquid product was collected and analyzed by GC and GC-MS.

The results were as follows: the coconut oil conversion was 100%, the hydrodecarbonylation plus hydrodecarboxylation selectivity was 86.5%, and the ratio of odd-numbered paraffins product to even-numbered paraffins product was 6.4. The yield of C₁₁-C₂₄ paraffins was 76.8wt.%, and the isomers content of C₁₁-C₂₄ paraffins was 71.6wt.%. The results are listed in Table 1.

### Comparative Example 7

A single-step process for producing second generation biodiesel and/or biojet from rice bran oil over Pt/ZSM-22 catalyst is described.

Refined rice bran oil (produced by Southseas Oils & Fats Industrial (Chiwan) Limited) up to National Standard GB19112-2003) with a fatty acid composition of 12.5% palmitic, 2.2% stearic, 45.3% oleic, 31.9% linoleic and 1.2% linolenic and an acidic value of 21 mgKOH.g-1 was fed to a fixed-bed reactor (10 mm i.d. and 600 mm in length) with a 1wt.%Pt/ZSM-22 catalyst (6.0 g, 10 mL, 10-20 meshes). The reaction was operated at a LHSV of 1 h⁻¹, 357 °C, 4.0 MPa and a H₂/oil ratio of 1765 NL/L. After 3 hours, the gas product was collected and analyzed by GC-TCD, while the liquid product was collected and analyzed by GC and GC-MS.

The results were as follows: the rice bran oil conversion was 100%, the hydrodecarbonylation plus hydrodecarboxylation selectivity was 71.4%, and the ratio of odd-numbered paraffins product to even-numbered paraffins product was 2.5. The yield of C₁₁-C₂₄ paraffins was 75.7wt.%, and the isomers content of C₁₁-C₂₄ paraffins was 84.3wt.%. The results are listed in Table 1.

### Comparative Example 8

A single-step process for producing second generation biodiesel and/or biojet from soybean oil over Pt/SAPO-11 and Pd/SAPO-11 is described.

Refined soybean oil (produced by COFCO, third grade, up to National Standard GB1535-2003) was fed to a fixed-bed reactor (10 mm i.d. and 600 mm in length) with a 0.5wt.%Pt/SAPO-11 catalyst (3.7 g, 5 mL, 10-20 meshes) and a 1wt.%Pd/SAPO-11 catalyst (3.9 g, 5 mL, 10-20 meshes). The reaction was operated at a LHSV of 1 h⁻¹, 350 °C, 4.0 MPa and a H₂/oil ratio of 1765 NL/L. After 3 hours, the gas product was collected and analyzed by GC-TCD, while the liquid product was collected and analyzed by GC and GC-MS.

The results were as follows: the soybean oil conversion was 100%, the hydrodecarbonylation plus hydrodecarboxylation selectivity was 78.4%, and the ratio of odd-numbered paraffins product to even-numbered paraffins product was 3.6. The yield of C₁₁-C₂₄ paraffins was 80.2wt.%, and the isomers content of C₁₁-C₂₄ paraffins was 82.7wt.%. The results are listed in Table 1.

### Example 9

A single-step process for producing second generation biodiesel and/or biojet from soybean oil over Pt-Ni/SAPO-11 catalyst is described.

Refined soybean oil (produced by COFCO, third grade, up to National Standard GB1535-2003) was fed to a fixed-bed reactor (10 mm i.d. and 600 mm in length) with a 1wt.%Pt-5wt.%Ni/SAPO-11 catalyst (7.6 g, 10 mL, 10-20 meshes). The reaction was operated at a LHSV of 0.6 h⁻¹, 339 °C, 4.0 MPa and a H₂/oil ratio of 1765 NL/L. After 3 hours, the gas product was collected and analyzed by GC-TCD, while the liquid product was collected and analyzed by GC and GC-MS.

The results were as follows: the soybean oil conversion was 100%, the hydrodecarbonylation plus hydrodecarboxylation selectivity was 77.5%, and the ratio of odd-numbered paraffins product to even-numbered paraffins product was 3.4. The yield of C₁₁-C₂₄ paraffins was 76.3wt.%, and the isomers content of C₁₁-C₂₄ paraffins was 73.6wt.%. The results are listed in Table 1.

### Example 10

A single-step process for producing second generation biodiesel and/or biojet from waste oil over Ni-Mo/SAPO-11 catalyst is described.

The purified waste oil with a fatty acid composition of 0.1 lauric, 0.1% myristic, 19.2% palmitic, 18.7% stearic, 41.5% oleic, 11.2% linoleic, 1.4% linolenic, 1.3% arachidic acid, 2.5% docosanoic acid and 1.2% lignoceric acid and an acidic value of 0.8 mgKOH.g-1 was fed to a fixed-bed reactor (10 mm i.d. and 600 mm in length) with a 5wt.%Ni-10wt.%Mo/SAPO-11 catalyst (8.2 g, 10 mL, 10-20 meshes). The reaction was operated at a LHSV of 0.6 h⁻¹, 355 °C, 4.0 MPa and a H₂/oil ratio of 1765 NL/L. After 3 hours, the gas product was collected and analyzed by GC-TCD, while the liquid product was collected and analyzed by GC and GC-MS.

The results were as follows: the waste oil conversion was 99.8%, the hydrodecarbonylation plus hydrodecarboxylation selectivity was 51.7%, and the ratio of odd-numbered paraffins product to even-numbered paraffins product was 1.1. The yield of C₁₁-C₂₄ paraffins was 79.8wt.%, and the isomers content of C₁₁-C₂₄ paraffins was 61.9wt.%. The results are listed in Table 1. The liquid product distribution is given in Table 2.

### Example 11

A single-step process for producing second generation biodiesel and/or biojet from animal fat over Ni-Mo/SAPO-11 catalyst is described.

The lard and tallow fat mixture (produced by Tianjin Lihongde Lipid Co., Ltd., up to National Standard GV10145-2005) with a fatty acid composition of 21.2% palmitic, 18.5% stearic, 45.5% oleic and 11.2% linoleic and a acidic value of 0.3 mg_{KOH}.g⁻¹ was fed to a fixed-bed reactor (10 mm i.d. and 600 mm in length) with a 5wt.%Ni-10wt.%Mo/SAPO-11 catalyst (8.2 g, 10 mL, 10-20 meshes). The reaction was operated at a LHSV of 0.6 h⁻¹, 365 °C, 4.0 MPa and a H₂/oil ratio of 1765 NL/L. After 3 hours, the gas product was collected and analyzed by GC-TCD, while the liquid product was collected and analyzed by GC and GC-MS.

The results were as follows: the animal fat conversion was 97.6%, the hydrodecarbonylation plus hydrodecarboxylation selectivity was 52.6%, and the ratio of odd-numbered paraffins product to even-numbered paraffins product is 1.1. The yield of C₁₁-C₂₄ paraffins was 79.2wt.%, and the isomers content of C₁₁-C₂₄ paraffins was 62.1wt.%. The results are listed in Table 1.

**Table 1. Reaction results of examples**

| Example | Conversion^{[a]} (%) | H&H selectivity^{[b]} (%) | P_{Odd/Even}^{[c]} | Y_{C11-C24} ^{[d]} (wt.%) | C_{*i*-C11-C24}^{[e]} (wt.%) |
|---|---|---|---|---|---|
| Comparative example | 100 | 28.8 | 0.4 | 80.2 | 86.2 |
| Example 2 | 100 | 70.6 | 2.4 | 81.4 | 66.3 |
| Example 3 | 99.2 | 57.5 | 1.4 | 79.8 | 81.5 |
| Example 4 | 100 | 50.3 | 1.0 | 77.5 | 73.9 |
| Example 5 | 99.0 | 52.7 | 1.1 | 79.6 | 66.2 |
| Example 6 | 100 | 86.5 | 6.4 | 76.8 | 71.6 |
| Example 7 | 100 | 71.4 | 2.5 | 75.7 | 84.3 |
| Example 8 | 100 | 78.4 | 3.6 | 80.2 | 82.7 |
| Example 9 | 100 | 77.5 | 3.4 | 76.3 | 73.6 |
| Example 10 | 99.8 | 51.7 | 1.1 | 79.8 | 61.9 |
| Example 11 | 97.6 | 52.6 | 1.1 | 79.2 | 62.1 |
| [a] Feedstock conversion, [b] Hydrodecarbonylation plus hydrodecarboxylation selectivity, [c] Ratio of odd-numbered paraffins to even-numbered paraffins, [d] Yield of C11-C24 paraffins, [e] C11-C24 iso-paraffins content. | | | | | |

**Table 2. The liquid product distribution in example 10.**

| Product | Content (%) |
|---|---|
| Undecane | 0.03 |
| Dodecane | 0.03 |
| Tridecane | 0.04 |
| Tetradecane | 0.04 |
| Pentadecane | 9.62 |
| Hexadecane | 9.43 |
| Heptadecane | 37.1 |
| Octadecane | 35.2 |
| Nonadecane | 0.62 |
| Eicosane | 0.59 |
| Heneicosane | 1.23 |
| Docosane | 1.18 |
| Tricosane | 0.59 |
| Tetracosane | 0.57 |
| ≤C10 paraffins | 1.89 |
| No paraffin type product | 0.8 |
| Total C11-C24 iso-paraffins | 61.9 |

Examples of the present invention provide a process for producing hydrocarbon product, for example product for use as, or as a component in, biodiesel and/or biojet. In examples, the main components of the hydrocarbon product are iso-paraffins. In examples, the hydrocarbon products are produced from a feedstock including a lipid, for example a fatty acid oil. In examples of the present process, catalytically hydrogenating, deoxygenating and hydroisomerizing the lipid feedstock to paraffins may be carried out in a single step. A character of examples of the process is that the oxygen atoms of the lipid are mainly converted to CO and CO₂, and less are converted to H₂O. Examples of the present single-step process can produce biodiesel and/or biojet with a high cetane value, low freezing point, low aromatics and low sulfur. More particularly, the hydrogen consumption of examples of the single-step process is less than that of a conventional two-step hydrogenation process.

It will be understood that the present invention has been described above purely by way of example, and modification of detail can be made within the scope of the invention.

## Claims

1. A process for producing a hydrocarbon product, the process comprising contacting a feedstock with a catalyst composition in the presence of hydrogen, the feedstock including a lipid, and the catalyst composition being active for conversion of the lipid to the hydrocarbon product in a single step, wherein the catalyst composition effects hydrogenation, deoxygenation and hydroisomerization of the lipid,
wherein the hydrocarbon product includes greater than 70 % measured by weight of C₁₁-C₂₄ alkanes based on the weight of the lipid, and the content of branched alkanes of the C₁₁-C₂₄ alkanes is greater than 60 % measured by weight of branched C₁₁-C₂₄ alkanes based on the weight of the C₁₁-C₂₄ alkanes, wherein the catalyst composition comprising an M1-[Sup] catalyst, where M1 is an active metal and [Sup] comprises an acidic support, wherein the acidic support includes a zeolite and/or SAPO and further comprises a refractory inorganic oxide,
wherein the amount of metal M1 present in the acidic support where M1 comprises cobalt, nickel, molybdenum or tungsten is between from about 1 to 30 wt.% based on the weight of the catalyst;
wherein the acidic support comprises one or more selected from the group comprising SAPO-11, SAPO-41, ZSM-5, ZSM-22, ZSM-23, ZSM-48 or mixtures thereof.

2. The process according to claim 1, wherein the acidic support comprises SAPO-11, ZSM-22 or mixtures thereof.

3. The process according to any preceding claim, wherein the catalyst composition includes at least 70 wt.% of [Sup] based on the weight of the catalyst.

4. The process according to any preceding claim, wherein the refractory inorganic oxide is selected from the group comprising alumina and silica, or mixtures thereof.

5. The process according to any preceding claim, wherein the refractory inorganic oxide is present in an amount of between from 20 to 60wt.% based on the weight of the catalyst.

6. The process according to any preceding claim, wherein the lipid feedstock comprises a triglyceride, diglyceride, monoglyceride, and/or fatty acid ester.

7. The process according to any preceding claim, wherein the lipid is selected from the group comprising vegetable oil, animal fat, and waste oil or mixtures thereof.

8. The process according to any preceding claim, wherein the temperature of the catalytic conversion is between from about 250 ºC to 450 ºC.

9. The process according to any preceding claim, wherein the pressure of the catalytic conversion is between from 1000 and 10000 kPa.

10. The process according to any preceding claim, wherein the feedstock comprises an oil and the hydrogen/oil ratio is in the range of from 300 to 3000 NL/L.

11. The process according to any preceding claim, wherein the liquid hourly space velocity of the fluid feedstock is from about 0.1 to 5.0 h⁻¹.

12. The process according to any preceding claim, wherein substantially all of any separation of products from the process is carried out after the conversion of the lipid to the hydrocarbon product.

13. The process according to any preceding claim, wherein the catalytic conversion is carried out in a reactor wherein the reactor comprises a fixed-bed reactor.

14. The process according to any preceding claim, wherein the catalytic conversion is carried out in a reactor system, the reactor system comprising one or more reactor elements.

15. The process according to claim 14, wherein the reactor or a reactor element of the system contains a further catalyst composition, wherein the further catalyst composition comprises an M2-[Sup₁] catalyst, where M2 is an active metal and [Sup₁] comprises an acidic support.

16. The process according to claim 15, wherein M2 is the same as M1and/or wherein [Sup₁] is the same as [Sup].

17. The process according to any of claim 14 to 16, wherein the reactor system comprises two or more reactor elements, the two or more reactor elements containing the same or different catalyst compositions.

## Patentansprüche

1. Ein Verfahren zur Herstellung eines Kohlenwasserstoffprodukts, wobei das Verfahren das Inkontaktbringen eines Ausgangsmaterials mit einer Katalysatorzusammensetzung in Gegenwart von Wasserstoff umfasst, wobei das Ausgangsmaterial ein Lipid umfasst, und die Katalysatorzusammensetzung für die Umwandlung des Lipids in das Kohlenwasserstoffprodukt in einem einzigen Schritt aktiv ist, wobei die Katalysatorzusammensetzung eine Hydrierung, Deoxygenierung und Hydroisomerisierung des Lipids bewirkt, wobei das Kohlenwasserstoffprodukt mehr als 70 Masse-% C₁₁-C₂₄-Alkane, bezogen auf das Gewicht des Lipids, umfasst, und der Gehalt an verzweigten Alkanen der C₁₁-C₂₄-Alkane größer als 60 Masse-%, bezogen auf das Gewicht der C₁₁-C₂₄-Alkane, ist, wobei die Katalysatorzusammensetzung einen M1-[Sup]-Katalysator umfasst, wobei M1 ein aktives Metall ist und [Sup] einen sauren Träger umfasst, wobei der saure Träger einen Zeolith und/oder SAPO umfasst und ferner ein hitzebeständiges anorganisches Oxid umfasst,
wobei die Menge an Metall M1, die in dem sauren Träger vorhanden ist, zwischen etwa 1 bis 30 Gew.-% liegt, bezogen auf das Gewicht des Katalysators, wobei M1 Kobalt, Nickel, Molybdän oder Wolfram umfasst;
wobei der saure Träger einen oder mehrere umfasst, ausgewählt aus der Gruppe, die SAPO-11, SAPO-41, ZSM-5, ZSM-22, ZSM-23, ZSM-48 oder Gemische davon umfasst.

2. Das Verfahren nach Anspruch 1, wobei der saure Träger SAPO-11, ZSM-22 oder Gemische davon umfasst.

3. Das Verfahren nach einem vorhergehenden Anspruch, wobei die Katalysatorzusammensetzung mindestens 70 Gew.% [Sup] bezogen auf das Gewicht des Katalysators umfasst.

4. Das Verfahren nach einem vorhergehenden Anspruch, wobei das hitzebeständige anorganische Oxid ausgewählt ist aus der Gruppe, die Aluminiumoxid und Siliciumdioxid, oder Mischungen davon, umfasst.

5. Das Verfahren nach einem vorhergehenden Anspruch, wobei das hitzebeständige anorganische Oxid in einer Menge von zwischen 20 und 60 Gew.-%, bezogen auf das Gewicht des Katalysators, vorliegt.

6. Das Verfahren nach einem vorhergehenden Anspruch, wobei das Lipid-Ausgangsmaterial ein Triglycerid, Diglycerid, Monoglycerid, und/oder Fettsäureester umfasst.

7. Das Verfahren nach einem vorhergehenden Anspruch, wobei das Lipid ausgewählt ist aus der Gruppe umfassend Pflanzenöl, tierisches Fett, und Altöl oder Mischungen davon.

8. Das Verfahren nach einem vorhergehenden Anspruch, wobei die Temperatur der katalytischen Umwandlung zwischen etwa 250 °C und 450 °C liegt.

9. Das Verfahren nach einem vorhergehenden Anspruch, wobei der Druck der katalytischen Umwandlung zwischen 1000 und 10000 kPa liegt.

10. Das Verfahren nach einem vorhergehenden Anspruch, wobei das Ausgangsmaterial ein Öl umfasst und das Wasserstoff/Öl-Verhältnis im Bereich von 300 bis 3000 NL/L liegt.

11. Das Verfahren nach einem vorhergehenden Anspruch, wobei die flüssige stündliche Raumgeschwindigkeit des flüssigen Ausgangsmaterials von etwa 0,1 bis 5,0 h⁻¹ beträgt.

12. Das Verfahren nach einem vorhergehenden Anspruch, wobei im Wesentlichen die gesamte Abtrennung von Produkten aus dem Verfahren nach der Umwandlung des Lipids in das Kohlenwasserstoffprodukt durchgeführt wird.

13. Das Verfahren nach einem vorhergehenden Anspruch, wobei die katalytische Umwandlung in einem Reaktor durchgeführt wird, wobei der Reaktor einen Festbettreaktor umfasst.

14. Das Verfahren nach einem vorhergehenden Anspruch, wobei die katalytische Umwandlung in einem Reaktorsystem durchgeführt wird, wobei das Reaktorsystem ein oder mehrere Reaktorelemente umfasst.

15. Das Verfahren nach Anspruch 14, wobei der Reaktor oder ein Reaktorelement des Systems eine weitere Katalysatorzusammensetzung enthält, wobei die weitere Katalysatorzusammensetzung einen M2-[Sup₁]-Katalysator umfasst, wobei M2 ein aktives Metall ist und [Sup₁] einen sauren Träger umfasst.

16. Das Verfahren nach Anspruch 15, wobei M2 das Gleiche ist wie M1 und/oder wobei [Sup₁] das Gleiche ist wie [Sup].

17. Das Verfahren nach einem der Ansprüche 14 bis 16, wobei das Reaktorsystem zwei oder mehr Reaktorelemente umfasst, wobei die zwei oder mehr Reaktorelemente die gleichen oder unterschiedliche Katalysatorzusammensetzungen enthalten.

## Revendications

1. Procédé de production d'un produit hydrocarboné, le procédé comprenant la mise en contact d'une charge avec une composition catalytique en présence d'hydrogène, la charge comprenant un lipide, et la composition catalytique étant active pour la conversion du lipide en produit hydrocarbure en une seule étape, dans lequel la composition de catalyseur effectue l'hydrogénation, la désoxygénation et l'hydroisomérisation du lipide, dans lequel le produit hydrocarboné comprend plus de 70 % mesurés en poids d'alcanes C₁₁-C₂₄ sur la base du poids du lipide, et la teneur en alcanes ramifiés du C₁₁-C₂₄ d'alcanes est supérieure à 60 % mesurés en poids d'alcanes ramifiés en C₁₁-C₂₄ par rapport au poids des alcanes en C₁₁-C₂₄,
dans lequel la composition catalytique comprenant un catalyseur M1-[Sup], où M1 est un métal actif et [Sup] comprend un support acide, dans lequel le support acide comprend une zéolite et/ou SAPO et comprend en outre un oxyde inorganique réfractaire, dans lequel la quantité de métal M1 présent dans le support acide où M1 comprend du cobalt, du nickel, du molybdène ou du tungstène est compris entre environ 1 et 30 % en poids sur la base du poids du catalyseur;
dans lequel le support acide comprend un ou plusieurs éléments choisis dans le groupe comprenant SAPO-11, SAPO-41, ZSM-5, ZSM-22, ZSM-23, ZSM-48 ou leurs mélanges.

2. Procédé selon la revendication 1, dans lequel le support acide comprend SAPO-11, ZSM-22 ou leurs mélanges.

3. Procédé selon l'une quelconque des revendications précédentes, dans lequel la composition de catalyseur comprend au moins 70% wt% de [Sup] sur la base du poids du catalyseur.

4. Procédé selon l'une quelconque des revendications précédentes, dans lequel l'oxyde inorganique réfractaire est choisi dans le groupe comprenant l'alumine et la silice, ou leurs mélanges.

5. Procédé selon l'une quelconque des revendications précédentes, dans lequel l'oxyde inorganique réfractaire est présent en une quantité comprise entre 20 et 60 % en poids sur la base du poids du catalyseur.

6. Procédé selon l'une quelconque des revendications précédentes, dans lequel la charge lipidique comprend un triglycéride, un diglycéride, un monoglycéride et/ou un ester d'acide gras.

7. Procédé selon l'une quelconque des revendications précédentes, dans lequel le lipide est choisi dans le groupe comprenant l'huile végétale, la graisse animale et l'huile usée ou leurs mélanges.

8. Procédé selon l'une quelconque des revendications précédentes, dans lequel la température de la conversion catalytique est comprise entre environ 250°C et 450°C.

9. Procédé selon l'une quelconque des revendications précédentes, dans lequel la pression de la conversion catalytique est comprise entre 1000 et 10000 kPa.

10. Procédé selon l'une quelconque des revendications précédentes, dans lequel la charge d'alimentation comprend une huile et le rapport hydrogène/huile est dans la plage de 300 à 3000 NL/L.

11. Procédé selon l'une quelconque des revendications précédentes, dans lequel la vitesse spatiale horaire liquide de la charge d'alimentation fluide est d'environ 0,1 à 5,0 h⁻¹.

12. Procédé selon l'une quelconque des revendications précédentes, dans lequel la quasi-totalité de toute séparation de produits du procédé est effectuée après la conversion du lipide en produit hydrocarboné.

13. Procédé selon l'une quelconque des revendications précédentes, dans lequel la conversion catalytique est effectuée dans un réacteur dans lequel le réacteur comprend un réacteur à lit fixe.

14. Procédé selon l'une quelconque des revendications précédentes, dans lequel la conversion catalytique est effectuée dans un système de réacteur, le système de réacteur comprenant un ou plusieurs éléments de réacteur.

15. Procédé selon la revendication 14, dans lequel le réacteur ou un élément de réacteur du système contient une autre composition de catalyseur, dans lequel l'autre composition de catalyseur comprend un catalyseur M2-[Sup₁], où M2 est un métal actif et [Sup₁] comprend un support acide.

16. Procédé selon la revendication 15, dans lequel M2 est identique à M1 et/ou dans lequel [Sup₁] est identique à [Sup].

17. Procédé selon l'une quelconque des revendications 14 à 16, dans lequel le système de réacteur comprend deux éléments de réacteur ou plus, les deux éléments de réacteur ou plus contenant des compositions de catalyseur identiques ou différentes.
